Europäisches Patentamt
European Patent Office
Office européen des brevets

(11) Veröffentlichungsnummer: **0 286 033**
**A2**

# EUROPÄISCHE PATENTANMELDUNG

(21) Anmeldenummer: 88105314.4

(22) Anmeldetag: 31.03.88

(51) Int. Cl.4: **C12P 1/00 , A23K 1/00 , A01N 63/00 , //(C12P1/00, C12R1:00)**

(30) Priorität: **02.04.87 DE 3711054**

(43) Veröffentlichungstag der Anmeldung: **12.10.88 Patentblatt 88/41**

(84) Benannte Vertragsstaaten: **AT BE CH DE ES FR GB GR IT LI LU NL SE**

(71) Anmelder: **SOCOP NAHRUNGSMITTEL GMBH**
**Wallenroder Strasse 6**
**D-1000 Berlin 26(DE)**

(72) Erfinder: **Wank, Anna**
**An der Apostelkirche 5 III**
**D-1000 Berlin 30(DE)**

(74) Vertreter: **Rotter, Ulrich, Dipl.-Chem. Dr. et al**
**Pienzenauerstrasse 2**
**D-8000 München 80(DE)**

(54) **Biophysikalisch derivatisiertes Ascomycetes-, Schizomycetes- und Hefepräparat enthaltende Futtermittel und Pflanzenwuchsstoffe, sowie Verwendung der Präparate zur Hautbehandlung und probiotischen Aktivierung.**

(57) Ein biophysikalisch derivatisiertes Hefepräparat wird vorgeschlagen, das gegenüber Leberhomogenat atmungssteigernde Aktivität aufweist, einen L-Aminosäureoxidase-Hemmfaktor enthält und darüber hinaus durch wundheilende Aktivität und eine RES-aktivierende Wirkung ausgezeichnet ist. Das Verfahren zur Herstellung des Hefepräparats sieht die Behandlung einer Ausgangshefe, ggf. unter Zusatz eines flüssigen Trägers vor, bis eine leicht resuspendierbare Masse erhalten wird, wonach eine kurzzeitige Wärmeschockbehandlung erfolgt, bis durch Gärung gebildete Gase entweichen. Zusätzlich werden Futtermittel und Pflanzenwuchsstofflösungen mit Zusätzen dieser biophysikalisch derivatisierten Hefen vorgeschlagen.

EP 0 286 033 A2

# Biophysikalisch derivatisiertes Ascomycetes-, Schizomycetes-und Hefepräparat, Verfahren zu dessen Herstellung und dieses Präparat enthaltende Futtermittel und Pflanzenwuchsstoffe, sowie Verwendung der Präparate zur Hautbehandlung und probiotischen Aktivierung

Die Erfindung betrifft ein Präparat aus biophysikalisch derivatisierten Ascomycetes-oder Schizomycetes-Zellmaterialien und insbesondere ein biophysikalisch derivatisiertes Hefepräparat mit erhöhter Stoffwechselaktivität, Verfahren zur Herstellung dieser Präparate, diese enthaltende Futtermittel und Pflanzenwuchsstoffe sowie die Verwendung der Präparate zur Hautbehandlung und zur probiotischen Aktivierung.

Es ist bekannt, daß pflanzliche und tierische Organismen den Zellstoffwechsel beeinflussen und aktivieren können. Auf dieser Grundlage sind bereits zahlreiche Methoden zur Stoffwechselaktivierung vorgeschlagen worden. Nach der DE-PS 10 76 888 werden atmungsfördernde Wirkstoffe aus dialysierten Blutzellen oder Blutplasmen gewonnen; sie sollen die Sauerstoffaufnahme von Rattenleberhomogenat erhöhen. Wachstumsfördernde Produkte werden nach der US-PS 39 37 816 aus der roten Pulpa von Rindermilz erhalten und bewirken bei intravenöser Injektion bei Mäusen in relativ kurzer Zeit eine Gewichtszunahme der Milz. Angaben über die Atmungsaktivität oder die Beeinflussung der Atmungskettenphosphorylierung werden nicht gemacht.

Bekannt ist außerdem, daß therapeutisch wirksame Substanzen in einer Hefe angereichert werden können (vgl. auch DE-OS 33 41 840).

Algen, Pilze und Hefen mit einer erhöhten Stoffwechselaktivität dergestalt, daß sie bei Rattenleberhomogenat eine atmungssteigernde Wirkung entfalten, waren bisher nicht bekannt. Die herkömmlichen Hefen, wie zum Beispiel Bierhefen, Saccharomyces cerevisiae und Saccharomyces carlsbergiensis, Bäckerhefen (Saccharomyces), Candida, Torula u.a. verhalten sich gegenüber einem solchen Homogenat inert. Der Atmungssteigerungsfaktor liegt im wesentlichen bei eins.

Die Erfindung stellt erstmals ein stoffwechselaktives Präparat aus Ascomycetes-oder Schizomycetes-Zellmaterialien und insbesondere ein Hefepräparat bereit, das eine atmungssteigernde Aktivität an standardisiertem Rattenleberhomogenat aufweist, wenn man im Warburgversuch mißt. Der Atmungssteigerungsfaktor bei 37 °C gegenüber einer entsprechenden Vergleichsprobe ist im allgemeinen mindestens 2,0 und hat vorzugsweise einen Wert von 2,0 bis 10.

Der Begriff "Ascomycetes" soll in vorliegender Beschreibung die Schlauchpilze mit schlauchartigen Hyphen und Ascosporabildung, wozu auch die Hefen (Saccharomyces) gehören, und die hefeähnlichen Pilze umfassen, deren Sporenbildung noch unbekannt ist, z.B. die Gattungen Candida und Torulopsis. "Schizomycetes" sind hefeartige Bakterien. In der Beschreibung wird der Begriff "Pilze" abkürzend für die vorgenannten Gattungen benutzt.

"Hefepräparat" bedeutet in der vorliegenden Beschreibung Hefeganzzellen, Hefelösung, Hefesuspensionen, einschließlich Hefeextrakt.

Der Atmungssteigerungsfaktor für die stoffwechselaktiven Hefepräparate der Erfindung wird anhand der Wirkung auf Rattenleberhomogenat nach der Warburg-Methodik ermittelt und stellt das Verhältnis des von standardisiertem Rattenleberhomogenat zusätzlich veratmeten Sauerstoffs (Volumen), bezogen auf eine präparatfreie Kontrolle ansonsten gleicher Zusammensetzung, dar.

Die Messung der Atmungssteigerung und des Atmungssteigerungsfaktors erfolgt in einem Warburggefäß bestimmten Volumens, das mit einem Puffer (vorzugsweise pH 7,4, Sörensen-Puffer), einer bestimmten Menge an konditioniertem Rattenleberhomogenat und der Testsubstanz bzw. Testlösung entsprechend einem Füllplan gefüllt wird und in dem die Testsubstanz meistens über einen Zeitraum von 60 min. bei 37 °C auf das Homogenat einwirkt. Durch Manometerablesung wird der $O_2$-Verbrauch ermittelt, nachdem bei der Atmung entstehendes $CO_2$ quantitativ absorbiert worden ist. Der auf Normalbedingungen umgerechnete Sauerstoffverbrauch, dividiert durch den Verbrauch einer Kontrollprobe ohne Testsubstanz, ist unmittelbar ein Maß für den Atmungssteigerungsfaktor.

Die meßbare Atmungssteigerung bei den erfindungsgemäßen Ascomycetes-, Schizomycetes-und Hefeproduktion ist dramatisch, was anhand der folgenden Tabelle I mit einem Hefeprodukt nach Beispiel 1 ersichtlich wird.

## Tabelle I

| Nr. | Probe | Atmungssteigerungsfaktor | prozentuale Steigerung (%) |
|---|---|---|---|
| 1 | Gesamtlösung | 4,4 | 340 |
| 2 | Zentrifugenüberstand | 4,0 | 300 |
| 3 | Zentrifugenrückstand | 2,8 | 180 |
| 4 | Ausgangsmaterial | 1,0 | - |

Charakteristisch für die erfindungsgemäßen Ascomycetes-, Schizomycetes-und Hefeprodukte ist eine gezielte Abwandlung ihrer Biochemie, wodurch die Stoffwechselaktivität stark erhöht wird. Kriterien für die Beurteilung der erhöhten Stoffwechselaktivität sind neben der bereits erläuterten Atmungssteigerung von Organhomogenaten eine verbesserte Wundheilung, die positive Beeinflussung des reticuloendothelialen Systems und die wachstumstimulierende Wirkung, die bei Fischen, aber auch bei Warmblütern festzustellen ist. Die stoffwechselaktiven Ascomycetes-, Schizomycetes-und Hefepräparate der Erfindung beeinflussen auch das Wachstum normaler Hefen positiv, worauf die folgenden Versuche hinweisen:

## Tabelle II

| Hefe | Hefezellen Einsaat (Zellen/ 10 ml) | Aussaat (Zellen/ 10 ml) | WSF(%) | WSR(%) |
|---|---|---|---|---|
| a) Sabouraud-Maltose 2 % Bouillon Normalhefe | $2{,}0 \cdot 10^6$ | $1{,}0 \cdot 10^9$ | 500 | - |
| Normalhefe + 2 % Erf. Hefeprodukt | $2{,}0 \cdot 10^6$ | $1{,}5 \cdot 10^9$ | 750 | 50 |
| b) Sabouraud-Maltose 2 % Bouillon Normalhefe | $5{,}0 \cdot 10^6$ | $2{,}0 \cdot 10^8$ | 40 | - |
| Normalhefe + 2 % Erf. Hefeprodukt | $5{,}0 \cdot 10^6$ | $2{,}6 \cdot 10^8$ | 52 | 30 |

WSF = Wachstumssteigerungsfaktor
WSR = Wachstumssteigerungsrate
Normalhefe (a) = Saccharomyces cerevisiae
Normalhefe (b) = Saccharomyces carlsbergiensis

Eine ähnliche positive Beeinflussung des Wachstums wurde auch bei Bakterien wie Lactobacillus-und Bifidus-Arten festgestellt.

In Hinsicht auf die veränderte Biochemie der derivatisierten Ausgangsprodukte auf Basis von Ascomycetes-, Schizomycetes-und Hefezellen sind die unter definierten Versuchsbedingungen erhaltenen TLC-Diagramme von Lipiden, Aminosäuren und Nucleinsäuren-Komponenten im Vergleich zu den zur Derivatisierung verwendeten Ausgangszellen von Interesse.

Die erhaltenen zweidimensionalen TLC-Diagramme eignen sich zur Charakterisierung der derivatisierten Produkte und zu ihrer Unterscheidung von anders hergestellten Produkten.

Abbildung 1 zeigt ein zweidimensionales TLC-Diagramm für Lipide und andere Inhaltsstoffe einer derivatisierten Hefe. Hierzu wurde 1 g derivatisierte Hefe mit 6 ml $CHCl_3/CH_3OH$ ( 2 : 1) extrahiert. Der Extrakt wurde chromatografiert:

1. Fließmittel $CHCl_3 : CH_3OH : H_2O$
(65 : 25 : 4)

2. Fließmittel $C_6H_6 : C_4H_{10}O : C_2H_6O : CH_3COOH$
(65 : 40 : 1 : 0)
Benzol:Diäthyläther:Ethanol:Essigsäure

Zum Nachweis der getrennten Inhaltsstoffe werden die Platten zuerst unter einer UV-Analysenlampe bei 366 nm und 254 nm betrachtet und die Flecken werden markiert. Zum Nachweis von Lipiden werden die Platten mit Primulin-Reagenz besprüht und uanter der UV-Analysenlampe bei 366 nm ausgewertet.

Überraschenderweise wurde bei den meisten erfindungsgemäßen Ascomycetes-, Schizomycetes-und Hefepräparaten zusätzlich zu der atmungssteigernden Aktivität ein Hemmfaktor vorgefunden, der inhibierend auf die Oxidasen wirkt. Als Oxidasen seien besonders genannt die Aminosäureoxidasen [1.4.3.2]bzw. 3] und die Monoaminooxidasen [1.4.3.4] sowie die Diaminooxidasen [1.4.3.6].

Ausgelöst durch diesen Effekt wird vermutlich die Oxidation von L-Aminosäuren, die an sich durch L-Aminosäureoxidase [1.4.3.2.] katalysiert ist, gehemmt bzw. verlangsamt. L-Aminosäureoxidase ist in vielen Nährstoffverwertungssystemen wie Darmflora und Bodenkulturen enthalten. Direkte Auswirkung dieses Hemmfaktors ist eine prozentual höhere L-Aminosäure-Nutzung in der Nahrung, die einem solchen Nährstoffverwertungssystem zugeführt wird, mit dem Resultat, daß das Tier bzw. die Pflanze schneller wachsen kann.

Ob dieser Hemmfaktor unmittelbar mit der erhöhten Atmungsaktivität zusammenhängt oder zumindest korreliert werden kann, ist noch nicht gesichert.

Ohne sich auf einen Mechanismus hier eindeutig festzulegen, könnte die Wirkung dieses Hemmfaktors wie folgt erklärt werden: L-Aminosäureoxidase enthält als prosthetische Gruppe fest gebundenes FMN (Flavinmononucleotid). Bei der Oxidation von L-Aminosäuren wird deren Aminogruppe nach folgendem Schema oxidiert:

1) L-AS + $H_2O$ + Enzym-FMN$\rightarrow$ $\alpha$-Ketosäure + $NH_3$ + Enzym-FMN.$H_2$

2) Enzym-FMN.$H_2$ + $O_2$ $\rightarrow$ Enzym-FMN + $H_2O_2$

Der Sauerstoffverbrauch kann unter Berücksichtigung der gebildeten $H_2O_2$-Menge gemessen werden, da $H_2O_2$ in Wasser und 1/2 $O_2$ zerfällt. In Gegenwart von Katalase ist dieser Zerfall spontan und quantitativ, so daß die Gesamtreaktion der L-Aminosäureoxidation der Gleichung 3 entspricht

$$3)\quad R-\underset{\displaystyle NH_2}{\underset{|}{CH}}-COOH + 1/2\ O_2 \longrightarrow R-CO-COOH + NH_3$$

Ist in einem System L-Aminosäureoxidase gehemmt oder blockiert oder in anderer Weise immobilisiert, wird weniger Sauerstoff verbraucht als ohne diesen Hemmfaktor, so daß hierdurch mehr Aminosäuren und Proteine verfügbar bleiben, was zu einer gesteigerten und intensiveren Nährstoffausnutzung führen kann.

Zur Bestimmung des L-Aminosäureoxidase-Hemmfaktors (Hmf) der erfindungsgemäßen Ascomycetes-, Schizomycetes-und Hefepräparate werden L-Aminosäureoxidaselösung (1 mg/ml), Katalasesuspension, Puffer pH 7,8 (50 ml 0,2 M $Na_2P_2O_7$ + 37,5 ml 0,2 M HCl), L-Phenylalaninlösung (1 mmol/50 ml, Puffer pH 7,8) und Testlösung (Zentrifugat, $10^5$ g; bzw. Hefesuspension) nach einem bestimmten Füllplan in ein Warburggefäß gefüllt, so daß die Messungen sowohl die Umsetzung von L-Aminosäureoxidase und L-Phenylalanin als auch die Umsetzung des Enzyms mit Testlösung (ohne Phenylalanin) und des Enzyms mit Testlösung und Phenylalanin erfassen. Die Zylindereinsätze werden mit Filterpapierstreifen versehen, die mit 6 N KOH getränkt sind. Die Lösungen werden bei 37 °C äquilibriert, dann wird die Enzymlösung in den die Testlösung bzw. eine Blindlösung enthaltenden Hauptraum gegeben, um die Reaktion zu starten. Der

µl-Verbrauch an Sauerstoff kann dann in Abhängigkeit von der Zeit aufgetragen werden und ist ein unmittelbares Maß für den Hemmfaktor, wenn man mit dem Sauerstoffverbrauch der Blindversuche korreliert. Im allgemeinen erfolgt die zur Bestimmung des Hmf benutzte Ablesung nach 60 min.

Zur Auswertung bildet man die Summenkurve der Meßkurven "L-Aminosäureoxidase + Phenylalanin" und "L-Aminosäureoxidase + Hefelösung" - der Sauerstoffbedarf ist auf die in der Hefe selbst enthaltenen Aminosäuren zurückzuführen - und setzt dann die Summenkurve, die ein Maß für die theoretisch zu erwartende Sauerstoffaufnahme ist, in Beziehung zur Kurve "L-Aminosäureoxidase + L-Phenylalanin + Hefelösung". Im Fall der erfindungsgemäßen Asomycetes-, Schizomycetes-und Hefepräparate (biophy sikalisch derivatisiert) ergibt sich ein Minderverbrauch an Sauerstoff, der den prozentualen Hemmfaktor ausdrückt (z.B. 0,42 = 42 % Hemmung bzw. weniger Sauerstoffverbrauch). Verglichen mit den jeweiligen Ausgangszellmaterialien, die praktisch den theoretischen Summenkurven entsprechen, ist der Hmf beachtlich und erreicht Werte von bis zu 70 oder sogar 80 %.

Eine weitere überraschende Eigenschaft der meisten erfindungsgemäßen Ascomycetes-, Schizomycetes-und Hefepräparate ist ihre Wundheilungsaktivität, die sich bei der Heilung von Schnittwunden am Rücken von Ratten bewerten läßt. Die Spannung an der Wundstelle der behandelten Tiere war nach 6 Tagen deutlich größer als bei einer Kontrollgruppe. Auch bei der Prüfung am 21. Tag war der Vorsprung der behandelten Tiere vor der Kontrollgruppe noch meßbar.

Bei diesem Test wurden männlichen Ratten der Wistar-Rasse mit einem Körpergewicht von 210 - 230 g narkotisiert, und unter Narkose wurden die Rückenhaare geschoren. Dann wurde entlang der Mediallinie ein geradliniger Schnitt von ca. 4 cm Länge angebracht und im Abstand von jeweils 1 cm an drei Stellen zusammengenäht. Am 6., 11., 16. und 21. Tag nach dem Eingriff wurde die Wunde geöffnet, und es wurden 3 schmale Hautscheiben im rechten Winkel zur Wundlinie genommen, einschließlich der 1 cm Breite der Wundlinie. Anschließend wurde die Spannung gemessen, die beim Abziehen der verwendeten Partie entstand.

2,5 ml/kg des Zentrifugenüberstandes der Testlösung aus Beispiel 1 wurden am Tage des Wundschnittes und dann täglich verabreicht. Die Kontrollgruppe erhielt eine Applikation von 2,5 ml/kg physiologische Kochsalzlösung in ansonsten gleicher Weise. Pro Gruppe wurden bei jeder Untersuchung jeweils 25 Tiere getestet.

Man konnte beobachten, daß die Spannung der verwundeten Hautpartie bei den mit der Testlösung behandelten Tieren gleichmäßig anstieg. Bei jeder Tagesprüfung zeigten die behandelten Tiere eine höhere Spannung der Hautpartie als die Kontrollgruppe. Im Anfangsstadium am 6. Tag war der Unterschied zur Kontrollgruppe größer als 100 %. Die Resultate sind in Tabelle III zusammengefaßt.

## Tabelle III

| Zahl der Tage | Testgruppe+ | Kontrollgruppe |
|---|---|---|
| 6 | 215 ± 17 | 104 ± 33 |
| 11 | 493 ± 24 | 390 ± 23 |
| 16 | 840 ± 48 | 730 ± 30 |
| 21 | 1659 ± 52 | 1401 ± 60 |

+Testgruppe erhielt 2,5 ml Testlösung/kg Körpergewicht

Die merklich verbesserte Wundheilung kann so erklärt werden, daß die erfindungsgemäßen Ascomycetes-, Schizomycetes-und Hefepräparate die Gewebeatmung fördern und die metabolische Funktion erhöhen, so daß das Gewebe wesentlich schneller regeneriert wird.

Nicht nur die verletzte Haut, sondern auch gesunde, unverletzte Haut wird durch das erfindungsgemäße Präparat, z.B. die derivatisierte Hefe, günstig beeinflußt. Unter Einwirkung eines biophysikalisch derivatisierten Hefepräparates (Beispiel 1) wurde das Feuchtigkeitshaltevermögen menschlicher Haut normalisiert und

vielfach verbessert. Um diese Wirkung nachzuweisen, wurde der Wassergehalt der Haut vor und nach der Behandlung über mehrere Stunden bestimmt. Präparat enthaltende Formulierungen wurden auf eine trockene Haut aufgebracht, also auf eine Haut mit schlechtem Feuchtigkeitsausgleich. Auf die gleiche Haut wurde eine Kontrollprobe ohne Präparat, aber ansonsten gleicher Zusammensetzung aufgegeben. Im Ergebnis zeigte sich, daß der Wassergehalt erhöht und das Feuchtigkeitshaltevermögen der Haut praktisch verdoppelt wurde. In Fig. 2 sind die Verlaufkurven für behandelte und unbehandelte Haut wiedergegeben. Auf der X-Achse sind die Stunden nach Behandlung und auf der Y-Achse ist die Feuchtigkeit der Haut in Prozent angegeben.

Wie schon anhand der Wundheilung erläutert, wird die Hautelastizität bzw. die Hautspannung durch Formulierungen, die ein erfindungsgemäßes Pilz-oder Hefepräparat enthalten, verbessert, was man durch Resonanzfrequenzmessungen an menschlicher Haut gut verfolgen kann.

Ein weiteres Kriterium für die gesteigerte Stoffwechselaktivität der erfindungsgemäßen Ascomycetes-, Schizomycetes-und Hefepräparate ist die positive Beeinflussung des reticuloendothelialen Systems. Aufgrund ihrer Wirkung auf die durch Tryphanblau gehemmte Funktion des RES der Ratten nach der Kongorot-Methode kann für die neuen Präparate eine RES-aktivierende Wirkung festgestellt werden. Hierzu wurden männliche Wistarratten mit einem Körpergewicht von 210 - 230 g intravenös mit 1 ml/kg einer 1%igen Kongorotlösung injiziert; nach 4 Minuten und nach 60 Minuten wurde Blut abgenommen. Der Kongorot-Koeffizient ergab sich aus dem Verhältnis der dekadischen Extinktion bei 500 nm des zehnfach verdünnten Serums. Zur Hemmung der Funktion des RES-Systems wurden 10 ml/kg 1%ige Tryphanblau-Lösung intraperitoneal verabreicht. Über die Messung der dekadischen Extinktion bei 650 nm konnte die Hemmung als berichtigte dekadische Extinktion bei 500 nm errechnet werden. Die Tryphanblau-Verabreichung erfolgte 6,5 Stunden vor Untersuchung des RES-Systems mit Kongorot.

Im Test wurden 5 Stunden vor dieser Untersuchung 0,5 ml der Testlösung (Zentrifugenüberstand nach Beispiel 1) intraperitoneal verabreicht. Die Kontrollgruppe erhielt eine adäquate Menge physiologische Kochsalzlösung. Pro Gruppe wurden jeweils 25 Versuchstiere getestet.

Für die behandelte Gruppe wurde im Mittel ein Kongorot-Koeffizient von 9,4 und für die Kontrollgruppe ein Kongorot-Koeffizient von 16,1 gefunden, was als ein direkter Nachweis für die steigernde Wirkung der erfindungsgemäßen Ascomycetes-, Schizomycetes-und Hefepräparate auf die Funktion des RES-Systems zu betrachten ist, da die hemmende Wirkung durch Tryphanblau aufgehoben wird.

Die für die erfindungsgemäßen Ascomycetes-, Schizomycetes-und Hefepräparate nachgewiesenen erhöhten Stoffwechselaktivitäten sind insofern völlig überraschend, als die Ascomycetes-, Schizomycetes-und Hefezellen und ihre Zellpräparate nur durch physikalische Methoden behandelt werden. Diese Behandlung wird hier allgemein "Derivatisierung" genannt. Die Ascomycetes-, Schizomycetes-und Hefezellen können durch Kälteeinwirkung, Laserbestrahlung, Sonifizierung und andere biophysikalische Methoden sowie eine relativ kurzzeitige intensive Wärmebehandlung derivatisiert werden. Hierbei kommt es offensichtlich zu einer deutlichen Verkleinerung von Zellen und der Hefezellen vorzugsweise auf das 0,5fache oder weniger, während die Zellwände stärker werden, so daß das Verhältnis von mittlerem Zelldurchmesser/Zellwandstärke um einen Faktor geändert wird, der bevorzugt im Bereich von etwa 5 bis 20 liegt.

Bevorzugt enthält das Präparat der Ascomycetes-und Schizomycetes-Zellmaterialien zusätzlich eine kleinere Menge an Mitochondrienextrakt, durch den die wachstumfördernde Aktivität des Präparates noch zusätzlich gesteigert werden kann. Der Mitochondrienextrakt wird am besten vor oder während der biophysikalischen Behandlung zugesetzt. Mitochondrien (auch Chondriosomen genannt) sind formvariable, ca. 0,1 bis 1 µm breite und 1-5 µm lange Einschlüsse im Protoplasma der Zellen von Einzellern, Pflanzen und Tieren. Sie sind im Handel als pyrogenfreie Trockenpulver oder als Lösungen erhältlich, wie z.B. Mitochondyl-Pulver der Fa. Widmer AG, Schweiz, das ca. 2 % Stickstoff und einen Trockensubstanzgehalt von ca. 50 % aufweist. Dieses Handelprodukt wird in den Beispielen eingesetzt. Im allgemeinen werden die Mitochondrienextrakte hergestellt durch Zertrümmern der Zellen, meistens Hefezellen, und anschließende geeignete Rehydratations-, Suspendierungs-, Wasch-und Trockenschritte. Siehe z.B. P.M. Nossal, Austral. J. exp. Biol. med. Sci. 31, 583 (1965); M. Merkenschläger, K. Schloßmann, W. Kurz, Biochem. Z. 329, 332 (1957). Mitochondyl stellt ein Produkt aus extrahierten Hefezell-Mitochondrien, vermischt mit Puffersubstanzen, dar und wird als weißes Pulver direkt oder durch Zubereiten einer entsprechenden gepufferten Lösung, die auch Konservierungsstoffe enthält, zugesetzt. Bevorzugt wird eine Mitochondrienextraktmenge von etwa 0,05 bis 0,50 Gew.-% verwendet, ausgedrückt als Trockensubstanz und bezogen auf den Trockenfeststoffanteil der zu derivatisierenden Zellen.

Eine Vermehrung der Ascomycetes-, Schizomycetes-und Hefezellen findet unter den gegebenen Bedingungen während der Behandlung praktisch nicht mehr statt und ist auch nicht erwünscht.

Der bevorzugt verwendete Träger besteht aus:

a) einer geringen Menge (bevorzugt 0,2 bis 5,0 Gew.-%) bereits biophysikalisch derivatisierter Hefe und/oder Chondriosomen-Extrakt (bevorzugt 0,01 bis 0,1 Gew.-%), gewonnen aus der als Ausgangsmaterial verwendeten Hefe oder einer anderen Hefe,

b) Celluloseglykolaten, pyrogener Kieselsäure, Tranganth und/oder Carrageenen,

c) zwei vergärungsfähigen Mono-und/oder Disacchariden,

d) Konservierungsmitteln

e) Spurenelementen wie Mn, Co, Zn, Mg, als Sulfate oder Glukonate.

Die Starterhefe (a) dient als Anlasser für die Behandlung, der Zusatz (b) sorgt für eine bessere Verteilung der Hefe. Die Kohlenhydrate (c) schützen vor der Vergärung der zelleigenen Kohlenhydrate. Als Konservierungsmittel (d) dienen bevorzugt Parabene (4-Hydroxybenzoesäurealkylester), Kochsalz und Calciumchlorid. Sie schützen das Endprodukt von Anfang an vor der Einwirkung von Mikroorganismen. Der Zusatz von Spurenelementen (e) ermöglicht erwünschte Konzentrationsverschiebungen in der Hefezelle bzw. Hefezellsuspension.

Auch das Wachstum von Mikroorganismen z.B. Bifidus, Lactobacillus, Kokken, Bodenbakterien und Darmbakterien, wird durch die erfindungsgemäßen Ascomycetes-, Schizomycetes-und Hefeprodukte positiv beeinflußt.

Mit den erfindungsgemäßen biophysikalischen derivatisierten Ascomycetes-, Schizomycetes-und Hefepräparaten wird bei höheren Pflanzen eine merkliche Beeinflussung des Pflanzenwachstums, des Blütenstandes und der Fruchtbildung erzielt. Werden dem Gießwasser bis zu 1 % der flüssigen Ascomycetes-, Schizomycetes-und Hefepräparate nach den folgenden Beispielen 1 bis 6 hinzugefügt, wachsen Zierpflanzen wie Tagetes, Petunien, Lobelien, Chrysanthemen und Nelken innerhalb einer Testperiode von sechs Wochen gegenüber einer Vergleichs(Kontroll)probe mindestens doppelt so schnell mit ausgeprägter Blatt- und Blütenfülle. Ähnliche Verbesserungen werden auch bei Nutzpflanzen wie Erbsen, Sonnenblumen und Mais beobachtet, womit die hervorragende Eignung der erfindungsgemäßen Präparate als wachstumstimulierender Zusatz zu Gieß-und Bewässerungslösungen belegt wird. Diese Wirkung ist wahrscheinlich mit einer günstigen Beeinflussung der Mikroflora des Bodens zu erklären.

## Beispiel 1

30 Gewichtsteile 95%ige Trockenhefe (Saccharomyces cerevisiae) und 70 Gewichtsteile einer wäßrigen Lösung bzw. Suspension eines Trägers enthaltend (in Gewichtsprozenten) 0,5 % biophysikalisch derivatisierte Hefe, 0,03 % Chondriosomen-Extrakt, 0,6 % Celluloseglykolate, 0,1 % Parabene, 2 % Salz, insgesamt 0,1 % Glukose und Saccharose, 0,00001 % Spurenelemente, werden bei einer Temperatur von 15 - 25 °C mit einem herkömmlichen $CO_2$-Laser bestrahlt. Die Leistung des Lasers in der Größenordnung von 0,3 Watt ist völlig ausreichend. Seine Fokussierung erfolgt auf 0,05 - 5 $kW/cm^2$.

Für die Aufnahme der Suspension zur Bestrahlung mit dem $CO_2$-Laser findet eine Vorrichtung Anwendung, die aus zwei Gefäßen geeigneter Größe besteht, die an den unteren Enden durch ein aus Infrarotglas bestehendes Rohr miteinander verbunden sind. Dieses Rohr hat eine Länge von 20 - 50 cm und einen Innendurchmesser von 3 - 6 mm. 20 l der Suspension werden mit Reinststickstoff in das mit Stickstoff gespülte linke Gefäß der Vorrichtung eingedrückt. Nachdem der Laser mit dem Endoskop auf eine Punktgröße entsprechend dem Innendruchmesser des Verbindungsrohrs eingestellt worden ist, wird die Suspension mit Stickstoff aus dem einen Gefäß in das andere Gefäß gedrückt, wobei auch das zweite Gefäß unter Stickstoff gesetzt wird. Bei einer Leistung von 0,3 W und einer Fließgeschwindigkeit der Suspension von 20 - 200 ml/min. wird bis zur gewünschten, mikroskopisch überprüfbaren Zellverkleinerung behandelt. Anschließend wird die Suspension kurz (höchstens 25 min.) auf eine Temperatur über 50 °C erwärmt und unter Rühren bei der Endtemperatur gehalten.

Die auf 30 °C abgekühlte Suspension wird dann zur Konservierung mit 0,1 bis 0,2 Gew.-% eines Konservierungsmittels wie Sorbinsäure und/oder Nipagin versetzt.

Die morphologische Untersuchung der Zellen ergab eine Verkleinerung des mittleren Zelldurchmessers auf ca. 50 % und eine deutliche Verstärkung der Zellwände. Sowohl das Gesamtprodukt (Suspension) als auch die durch Zentrifugieren (10.000 Upm) erhältlichen Fraktionen, d.h. Zentrifugenüberstand bzw. Zentrifugenrückstand, erweisen sich im Warburg-Versuch als stoffwechselaktiv (im Gegensatz zur Ausgangshefe), wenn gemäß dem beschriebenen Test auf atmungssteigernde Wirkung bei Rattenleberhomogenat geprüft wurde. Der Atmungssteigerungsfaktor für das Gesamtprodukt und für den Zentrifugenüberstand war größer als 4 (siehe Tabelle I). Weitere Aktivitäten wurden im beschriebenen Wundheilungstest (Tabelle III) und im Test zur Aktivierung des RES-Systems nachgewiesen.

In einer Dosierung von 2 ‰ wurde das Gesamtprodukt bei der Vormast von 10 - 35 kg wiegenden

Ferkeln bzw. Absetzferkeln getestet (siehe Tabelle IV bzw. V). Die Verbesserung der täglichen Futteraufnahme war mit ca. 58 % bzw. 42 % deutlich größer als bei Anwendung handelsgängiger Futterzusatzpräparate, so daß eine Verbesserung der Futterverwertung von 16,7 bzw. 19,5 % resultierte. Die Ergebnisse sind im einzelnen in den folgenden Tabellen IV und V zusammengefaßt.

## Tabelle IV

Ferkelaufzucht (10 - 35 kg Lebendgewicht)

| | Kontrolle (ohne) | Handelspräparate A | Handelspräparate B | Testpräparat (2°/oo) |
|---|---|---|---|---|
| Tierzahl/ Versuchsgruppe | 24 | 24 | 24 | 24 |
| Anfangsgewicht (kg) | 10,2 | 10,1 | 10,3 | 10,2 |
| Versuchsdauer (Tage) | 35 | 35 | 35 | 35 |
| Gewicht am Versuchsende (kg) | 25,4 | 26,7 | 30,2 | 34,2 |
| Gewichtszunahme (kg) | 15,2 | 16,6 | 19,9 | 24,0 |
| tägliche Zunahme (g) | 434 | 474 | 569 | 686 |
| relative Differenz (%) | 100 | 109 | 131 | 158 |
| Futterverzehr/ Tier/Tag (g) | 908 | 971 | 1093 | 1194 |
| Futteraufwand/kg Zuwachs (kg) | 2,09 | 2,05 | 1,92 | 1,74 |
| relative Differenz (%) | 100 | 98 | 92 | 83,3 |
| Verbesserung: | | | | |
| tägliche Zunahme (%) | - | 9,21 | 30,9 | 57,9 |
| Futterverwertung (%) | - | 2 | 8 | 16,7 |

Die Handelspräparate waren A: CTC und B: bayo-nox (ebenso in Tabelle V)

## Tabelle V

Versuch mit Absetzferkeln
(Absetzalter 21 Tage, Lebendgewicht 5 - 12,5 kg)

| | Kontrolle (ohne) | Handelspräparate A | Handelspräparate B | Testpräparat (2°/oo) |
|---|---|---|---|---|
| Tierzahl/ Versuchsgruppe | 24 | 24 | 24 | 24 |
| Anfangsgewicht (kg) | 4,93 | 5,14 | 5,12 | 5,06 |
| Versuchsdauer (Tage) | 20 | 20 | 20 | 20 |
| Gewicht am Versuchsende (kg) | 10,2 | 11 | 11,9 | 12,9 |
| Gesamtgewichtszunahme (kg) | 5,23 | 5,84 | 6,78 | 7,45 |
| tägliche Zunahme (g) | 262 | 292 | 339 | 373 |
| relative Differenz (%) | 100 | 112 | 130 | 142 |
| Futterverzehr/ Tier/Tag (g) | 429 | 465 | 480 | 492 |
| Futteraufwand/kg Zuwachs (kg) | 1,64 | 1,59 | 1,42 | 1,32 |
| relative Differenz (%) | 100 | 97 | 86,3 | 80,5 |
| **Verbesserung:** | | | | |
| tägliche Zunahme (%) | - | 11,7 | 29,6 | 42,4 |
| Futterverwertung (%) | - | 3 | 13,7 | 19,5 |

Beispiel 2

100 l einer Suspension von Torulopsis utilis und Saccharomyces cerevisiae (2:8 Gemisch) mit einem Trockenfeststoffgehalt von insgesamt 20 % werden mit einer Mischung versetzt, die enthält:

1 kg Produkt aus Beispiel 1
0,05 kg Chondriosomen-Extrakt
0,1 kg Celluloseglykolat
0,1 kg Gummi arabicum

0,1 kg Glukose + Mannose
0,1 kg Parabene
1,5 kg eines Salzgemisches aus Kochsalz, $MnSO_4$ und $MgSO_4$ und
0,00001 % Spurenelemente

Die Suspension wird gerührt und bei 20 °C mit Ultraschall behandelt und während der Sonifizierung zunächst auf 45 °C und dann kurzzeitig (2 min.) bis auf 80 °C erhitzt.

Die abgekühlte Suspension wird mit 200 g Kieselsäure (Aerosol 300) und genügend Nipagin (Konservierungsmittel) in einer geeigneten Vorrichtung (z.B. Büchi-Apparatur) sprühgetrocknet, wobei pro Stunde ca. 1,5 kg festes Produkt anfallen.

Der Atmungssteigerungsfaktor bei Prüfung auf atmungssteigernde Aktivität an Rattenleberhomogenat war 5,1, getestet am flüssigen Produkt (vor Sprühtrocknen). Im Wundheilungsversuch wurde ein Vorsprung von ca. 100 % gegenüber einer Kontrollgruppe gefunden, wenn - wie beschrieben - die Spannung der Hautpartien gemessen wurde. Die Ausgangshefe war demgegenüber inaktiv.

Die erhöhte Stoffwechselaktivität des Präparates zeigte sich in Wachstumsversuchen an Fischen und Hühnern. Bereits sehr geringe Mengen des Zusatzes zum Normalfutter (Zusätze kleiner als 1 ‰) bewirkten einen überraschend hohen Wachstumseffekt, was sich in einer beträchtlichen Futterersparnis manifestierte. Die Tabelle VI und VII fassen die Ergebnisse zusammen.

## Tabelle VI

### Wachstumsversuche bei Schwertfischen

| | Kontrolle | Zusatz 0,80°/oo | Zusatz 0,95°/oo |
|---|---|---|---|
| mittleres Gewicht der Jungfische nach 47 Tagen | 1,9 | 3,7 | 4,1 |

Bei Versuchsdurchführung wurden 60 Jungfische am 15. Tag in die drei Gruppen unterteilt und in einer Wassermenge von 5 l bei einer mittleren Beckentemperatur von 24,5 °C zweimal wöchentlich gefüttert. Zwei Fischgruppen erhielten 0,8 bzw. 0,95 ‰ Zusatz des Präparates aus Beispiel 2, verteilt auf 3 Gaben pro Woche.

Der Gewichtszuwachs ist außerordentlich und belegt den wachstumsfördernden Effekt des Präparates.

Das sprühgetrocknete Produkt aus Beispiel 2 wurde als Zusatz zum Futtermittel von Harvard-Hühnern getestet. Eine aus 3.600 Hühnern bestehende Versuchsgruppe wurde 60 Tage unter 0,1 Gew.-% Zusatz-Anreicherung gefüttert. Eine aus 3.600 Hühnern bestehende Kontrollgruppe erhielt Futter ohne Zusatzstoff. Körpergewicht von je 20 Hühnern und Sterblichkeit wurden ermittelt (Tabelle VII).

## Tabelle VII

### Fütterungsversuche bei Hühnern

| Tag Nr.: | 1 | 10 | 20 | 30 | 40 | 50 | 60 |
|---|---|---|---|---|---|---|---|
| Gewicht der Versuchsgruppe (g) | 34 | 209 | 568 | 1110 | 1590 | 2001 | 2090 |
| Gewicht der Kontrollgruppe (g) | 39 | 246 | 550 | 1100 | 1510 | 1890 | 1990 |

Hieraus ergab sich ein Nahrungsverbrauch (kg) pro kg Körpergewicht für die Versuchsgruppe von 2,27 und für die Kontrollgruppe von 2,65. Die Sterblichkeit bei der Versuchsgruppe war 4,9 %, die der

Kontrollgruppe 7,7 %. Die verbesserte Futterverwertung entspricht einer Futterersparnis von ca. 15 %.

Beispiel 3

10 kg Preßhefe (Saccharomyces cerevisiae) werden in einer geeigneten Kühlvorrichtung auf eine Temperatur unter -13 °C abgekühlt und etwa 4 Std. bei dieser niedrigen Temperatur konditioniert. Anschließend werden zu dem Produkt 500 ml einer wäßrigen Suspension gegeben, die 15 g Fructose und Saccharose, 40 g feinteilige Kieselsäure ($SiO_2$), 50 g Präparat aus Beispiel 2 (Basis Feststoffprodukt), 15 g Parabene und 160 g Kochsalz enthält; das Gemisch wird zunächst innig vermischt und auf Raumtemperatur gebracht (ca. 23 °C), wobei sich eine flüssige Mischung bildet, in der die Hefeteilchen leicht suspendiert werden. Das Gemisch wird dann schnell in der Weise erwärmt, daß der Temperaturanstieg ca. 5°/3 min. beträgt. Die Mischung wird bis auf eine Temperatur zwischen 45 °C und 75 °C erwärmt, während ständig gerührt wird.

Es bildet sich eine dünnflüssige Hefesuspension mit leicht resuspendierbaren Teilchen, die als Gesamtlösung, aber auch als Zentrifugat (Überstand bzw. Rückstand) einen Atmungssteigerungsfaktor von 4,8 aufweist.

Beispiel 4

10 kg Preßhefe (Saccharomyces cerevisiae) werden auf eine Temperatur unter -15 °C gebracht. Nach 24 Std. werden hinzugefügt: 10 g Parabene, 5 g Sorbinsäure, 170 g NaCl als Konservierungsmittel und 10 g Saccharose, 15 g $SiO_2$, 10 g Celluloseglykolat und 0,8 mg Spurenelemente (Mn, Co, Zn, Mg).

Nachdem das Gemisch Raumtemperatur (25 °C erreicht hat, wird es unter Rühren in die in Beispiel 1 beschriebenen Laser-Apparatur gebracht und mit Laserstrahlung solange behandelt, bis die Hefeteilchen auf mindestens 50 % ihres Durchmessers vor der Lasereinwirkung geschrumpft sind. Anschließend wird unter weiterem Rühren noch kurz erwärmt (höchstens 20 min.), bis eine Temperatur zwischen 40° und 80 °C erreicht ist.

Der Atmungssteigerungsfaktor dieses Produktes ist 5,1. Nach Konservieren der Suspension wird sprühgetrocknet. Das Produkt kann als biophysikalisch derivatisierte Starterhefe in den Beispielen 1 - 3 eingesetzt werden. Das erhaltene Produkt diente für Fütterungsversuche, wobei die stoffwechselsteigernde Aktivität derjenigen des Beispiels 2 vergleichbar war, wenn als Futtermittelzusatz bei Harvard-Hühnern getestet wurde. Diese Aktivität läuft in der Praxis auf eine merkliche Futterersparnis und stark verkürzte Fütterungszeit hinaus, was sich insbesondere bei Großversuchen auszahlt.

Auch bei der Fütterung von Pelztieren wie Chinchillas erwies sich das Präparat als außerordentlich nützlich. Das Haarkleid ist nach kurzer Zeit der Anwendung des Präparates glatter und glänzender. Das Fell älterer Zuchttiere ist noch verwendbar, wenn diese das aus Hefe hergestellte Präparat einige Zeit erhalten haben.

Beispiel 5

500 kg 20%ige Hefe (Saccharomyces), sogenannte 20 er Hefe, wurden bei 20 °C mit 0,2 kg Parabene, 0,03 kg Sorbinsäure, 2 kg NaCl, 0,2 kg Saccharose, 0,5 kg $SiO_2$, 0,3 kg Celluloseglykolat, 0,5 kg Mitochondrienextrakt und 10 mg Spurenelementen versetzt und mit einem Ultraturax als Rührer so stark gerührt, daß sich die Mischung rasch erwärmte. Bei 55° bis 60 °C wurde noch etwa 10 min. gerührt und dann abgekühlt. Bei Fütterungsversuchen wurden ver gleichbare Testergebnisse erzielt wie in den Beispielen 1 und 2.

Beispiel 6

50 kg 20 er Hefe (Saccharomyces) wurden bei 20 °C mit 20 g Glucose und Fruktose (10 : 1), 40 g Alginat, 30 g Mitochondrienextrakt, 180 g NaCl, 10 g Natriumascorbat als Konservierungsmittel und 1,5 mg Spurenelementen versetzt und mit dem Ultraturrax unter gleichzeitiger Sonifizierung gerührt, wobei sich die Mischung ohne externe Wärmezufuhr auf 55 °C erwärmte und bei 52 - 60 °C kurze Zeit, jedoch nicht

länger als 20 min. gehalten wurde. Die abgekühlte Mischung wurde zur Steigerung der Joghurtproduktion eingesetzt, wobei den Kulturen weniger als 0,1 Gew.-%, bezogen auf das Gewicht des Gesamtmediums, hinzugefügt wurde. Das Produkt war auch für andere Belange in der Molkereiindustrie verwendbar, da es das Wachstum der entsprechenden Mikroflora beschleunigt.

## Ansprüche

1. Präparat aus biophysikalisch derivatisierten Ascomycetes-oder Schizomycetes-Zellmaterialien, mit atmungssteigernder Aktivität, die im Warburg-Gefäß an standardisiertem Rattenleberhomogenat meßbar ist und als das vom Homogenat zusätzlich veratmete Sauerstoffvolumen ausgedrückt wird.

2. Biophysikalisch derivatisiertes Hefepräparat mit atmungssteigernder Aktivität, die im Warburg-Gefäß an standardisiertem Rattenleberhomogenat meßbar ist und als das vom Homogenat zusätzlich veratmete Sauerstoffvolumen ausgedrückt wird.

3. Präparat nach Anspruch 1 oder 2, dadurch gekennzeichnet, daß der Atmungssteigerungsfaktor bei 37 °C mindestens 2,0 beträgt, bezogen auf das Homogenat unter ansonsten gleichen Versuchsbedingungen.

4. Präparat nach Anspruch 1 oder 2, dadurch gekennzeichnet, daß der Atmungssteigerungsfaktor bei 37 °C einen Wert von 2,0 bis 10 und vorzugsweise einen Wert von 2,5 bis 5,5 aufweist.

5. Präparat nach Anspruch 1 oder 2, dadurch gekennzeichnet, daß es außerdem einen L-Aminosäureoxidase-Hemmfaktor enthält, meßbar im Warburg-Gefäß an einer standardisierten Testlösung mit dem Enzym und Phenylalanin als L-Aminosäure und ausgedrückt als Verhältnis des Sauerstoffverbrauchs mit und ohne Hefepräparat.

6. Präparat nach Anspruch 5, dadurch gekennzeichnet, daß der L-Aminosäureoxidase-Hemmfaktor einen Wert im Bereich von etwa 20 bis 80 % und vorzugsweise einen Wert im Bereich von 30 bis 60 % hat.

7. Präparat nach Anspruch 1 oder 2, dadurch gekennzeichnet, daß es wundheilende Aktivität aufweist.

8. Präparat nach Anspruch 1 oder 2, dadurch gekennzeichnet, daß es zusätzlich eine RES-aktivierende Wirkung zeigt.

9. Präparat nach Anspruch 1 oder 2, dadurch gekennzeichnet, daß es das Wachstum von Hefen und Mikroorganismen, wie z.B. Bifidus, Lactobacillus, Kokken und Bodenbakterien positiv beeinflußt und damit indirekt auch das Wachstum von höheren Pflanzen und Algen fördert.

10. Präparat nach Anspruch 1 oder 2, dadurch gekennzeichnet, daß es zusätzlich aus tierischen und/oder pflanzlichen Zellen extrahierte Mitochondrien enthält.

11. Verfahren zur Herstellung des Präparates aus biophysikalisch derivatisierten Ascomycetes-oder Schizomycetes-Zellmaterialien mit atmungssteigernder Aktivität, dadurch gekennzeichnet, daß Ascomycetes-oder Schizomycetes-Zellen, gegebenenfalls unter Zusatz eines flüssigen Trägers durch Einwirkung von Laserstrahlung, Gefriertrocknung, Ultraschallbehandlung, kurzzeitige Druckbeaufschlagung und/oder Osmolyse solange behandelt werden, bis die Zellen in eine leicht resus pendierbare Masse umgewandelt worden sind, und die Suspension der kontrahierten Zellen einer kurzzeitigen intensiven Wärmeschockbehandlung unterzogen wird, bis durch Gärung gebildete Gase entweichen.

12. Verfahren zur Herstellung eines biophysikalisch derivatisierten Hefepräparates mit atmungssteigernder Aktivität, dadurch gekennzeichnet, daß eine Ausgangshefe, gegebenenfalls unter Zusatz eines flüssigen Trägers, durch Einwirkung von Laserstrahlung, Gefriertrocknung, Ultraschallbehandlung, kurzzeitige Druckbeaufschlagung und/oder Osmolyse solange behandelt wird, bis die Hefezellen in eine leicht resuspendierbare Masse umgewandelt worden sind, und die Suspension der kontrahierten Hefezellen einer kurzzeitigen intensiven Wärmeschockbehandlung unterzogen wird, bis durch Gärung gebildete Gase entweichen.

13. Verfahren nach Anspruch 11 oder 12, dadurch gekennzeichnet, daß die Wärmebehandlung die Zellen nicht länger als 25 min. bei einer Temperatur über 40 °C beläßt.

14. Verfahren nach Anspruch 11 oder 12, dadurch gekennzeichnet, daß die Wärmebehandlung direkt mit einem im Gegenstrom zugeleiteten Heißgas bewirkt wird.

15. Verfahren nach einem der Ansprüche 11 bis 14, dadurch gekennzeichnet, daß die biophysikalische Zellverkleinerung gleichzeitig mit der kurzzeitigen intensiven Wärmeeinwirkung ausgeführt wird.

16. Verfahren nach einem der Ansprüche 11 bis 15, dadurch gekennzeichnet, daß als flüssiger Träger eine wäßrige Lösung oder Suspension zugesetzt wird, die enthält:

1) 0,1 bis 0,5 % biophysikalisch derivatisierte Hefe, die entweder aus einer Hefe, die als Ausgangsmaterial dient und/oder aus einer anderen Hefe erhalten wurde, und 0,02 bis 0,4 % Chondriosomen-Extrakt,

2) 0,3 bis 5 % Celluloseglykolat, hochdisperse pyrogene Kieselsäure mit mehr als 99,8 % $SiO_2$ und einem Durchmesser von 7 bis 25 nm, Carrageen, Traganth oder ähnliche Füllstoffe,

3) 0,01 bis 0,3 % vergärbare Mono-und/oder Disaccharide,

4) Konservierungsmittel wie Parabene und Salze wie Kochsalz in ausreichender Konzentration,

5) Spurenelemente wie Mn, Co, Zn, Mg, vorzugsweise als Sulfate oder Glukonate.

17. Verfahren nach einem der Ansprüche 11 bis 16, dadurch gekennzeichnet, daß als Ausgangszellmaterial ein Stamm aus der Familie der Saccharomycetaceae eingesetzt wird.

18. Verfahren nach einem der Ansprüche 11 bis 17, dadurch gekennzeichnet, daß eine Trockenhefe mit einem HTS-Gehalt von 95 bis 98 % zusammen mit der 2-bis 4-fachen Menge eines flüssigen Trägers behandelt wird.

19. Futtermittel, gekennzeichnet durch ein herkömmliches Futter, dem bis zu 1 Gew.-% des biophysikalisch derivatisierten Präparates mit atmungssteigernder Aktivität nach einem der Ansprüche 1 bis 18 zugesetzt sind.

20. Pflanzenwuchsstofflösung aus Gießwasser und bis zu 2 Gew.-% des biophysikalisch derivatisierten Präparats nach einem der Ansprüche 1 bis 18.

21. Verwendung des biophysikalisch derivatisierten Präparats nach einem der Ansprüche 1 bis 10 zur Hautbehandlung oder Hautkonditionierung.

22. Verwendung nach Anspruch 21 zur Normalisierung und Steigerung des Feuchtigkeitshaltevermögens der Haut.

23. Verwendung nach Anspruch 21 zur Erhöhung der Elastizität der Haut.

24. Verwendung nach Anspruch 21 zur Wundheilung von Haut.

25. Verwendung nach Anspruch 21 in Kombination mit an sich bekannten Hautheilungs-und/oder Hautkonditionierungsmitteln wie Kollagen, Organextrakten, Pflanzenextrakten oder mehrwertigen Alkoholen mit 2 bis 8 Kohlenstoffatomen.

26. Verwendung des biophysikalisch derivatisierten Präparats nach einem der Ansprüche 1 bis 10 zur probiotischen Aktivierung von Molkereiprodukten, wobei das derivatisierte Zellmaterial mit ausgewählten natürlichen Mikroorganismen wachstumstimulierend zusammenwirkt.

27. Verwendung nach Anspruch 26 bei der Joghurterzeugung.

28. Verwendung nach Anspruch 26 zur Wachstumstimulierung von Lactobacillus bulgaricus.

29. Verwendung des biophysikalisch derivatisierten Präparats nach einem der Ansprüche 1 bis 10 zur probiotischen Aktivierung von Actinomyceten, insbesondere zur Aktivierung von Hefen.

30. Verwendung nach Anspruch 29 zur Aktivierung von Hefen aus der folgenden Gruppe: Aspergillus niger, Torula utilis, Neurospora crassa, Saccharomyces-Arten und japanische Ikashiokara-Hefe.

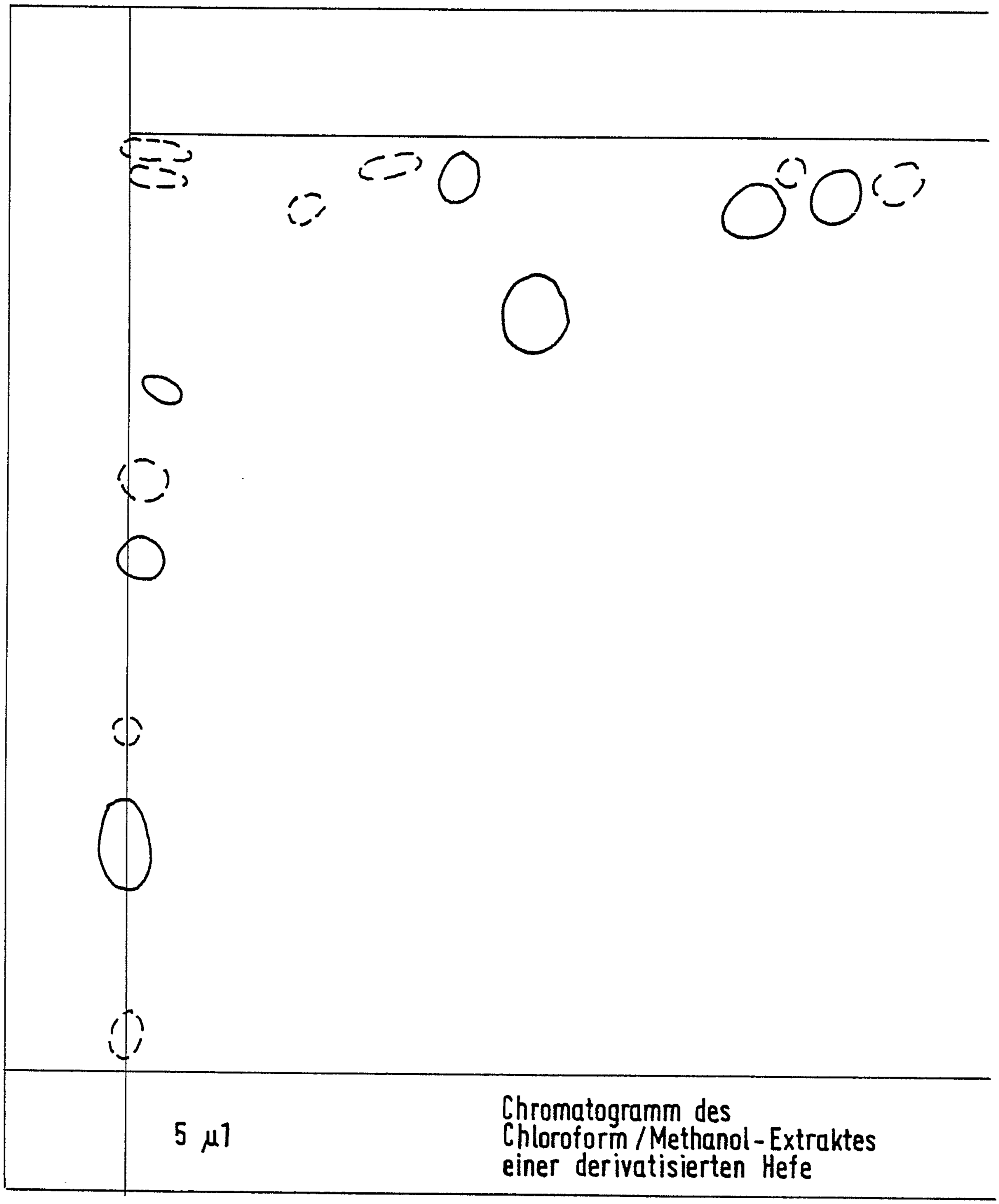
5 µl
Chromatogramm des
Chloroform / Methanol - Extraktes
einer derivatisierten Hefe

FIG. 1

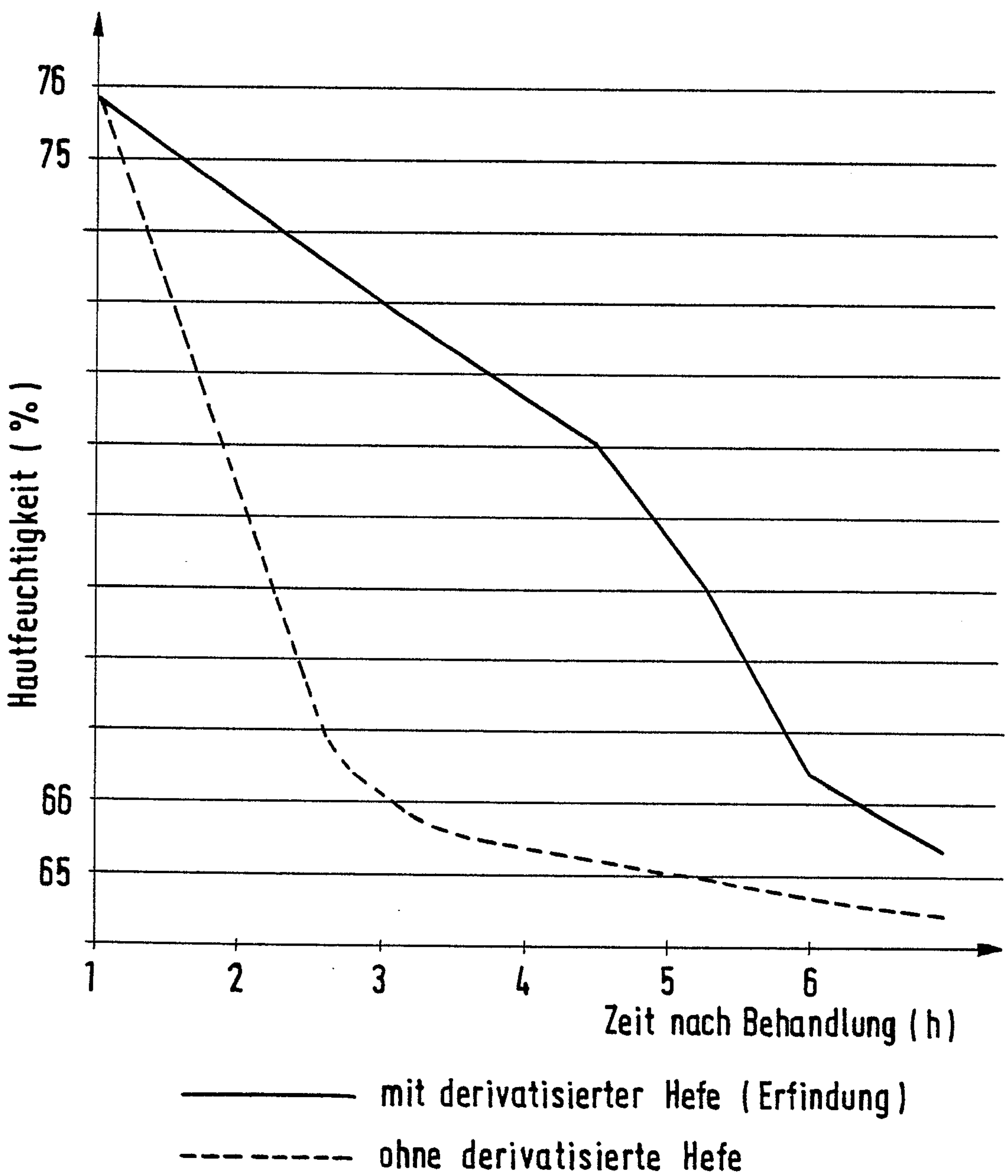

76
75
66
65
Hautfeuchtigkeit ( % )
1
2
3
4
5
6
Zeit nach Behandlung ( h )
mit derivatisierter Hefe ( Erfindung )
ohne derivatisierte Hefe


FIG. 2